# EUROPEAN PATENT APPLICATION

(11) **EP 4 654 125 A1**
(43) Date of publication of application: **26.11.2025**
(21) Application number: 24744764.2
(22) Date of filing: 03.01.2024
(51) Int. Cl.: G06T 7/00, G06T 7/11, G06T 5/80, G06V 20/69, G16H 30/40, G06T 5/70

(54) **METHOD AND DEVICE FOR ANALYZING PATHOLOGY SLIDE IMAGE**

(30) Priority: 19.01.2023 KR 20230008291; 24.05.2023 KR 20230067013
(71) Applicant: Lunit Inc., Seoul, 06241 (KR)
(72) Inventor: CHO, Soo Ick, Seoul 06241 (KR); JUNG, Won Kyung, Seoul 06241 (KR)
(74) Representative: AWA Sweden AB
(86) International application number: PCT/KR2024/000073
(87) International publication number: WO 2024/154982

(57) **Abstract**

A computing device according to an aspect includes: at least one memory; and at least one processor, wherein the processor is configured to divide a pathological slide image into a plurality of patches, determine whether or not at least one artifact is present in at least one of the plurality of patches, and perform an additional task on the basis of a result of the determination.

## Description

### Technical Field

The present disclosure relates to a method and device for analyzing a pathological slide image.

### Background Art

The field of digital pathology refers to a field that acquires histological information regarding corresponding patients or predicts prognosis of the patients by using whole slide images generated by scanning pathological slide images.

The pathological slide images may be acquired from stained tissue samples of subjects. For example, tissue samples may be stained by various staining methods, such as hematoxylin and eosin, trichrome, periodic acid schiff, autoradiography, enzyme histochemistry, immuno-fluorescence, and immunohistochemistry. The stained tissue samples may be used for histological and biopsy evaluations, and thus may operate as bases for determining whether or not to move on to molecular profile analysis to understand disease states.

Meanwhile, the quality of pathological slide images may be deteriorated due to various factors, and the degree of deterioration of the quality may vary. Accordingly, there is an increasing demand for technologies that perform different analysis procedures on images according to the quality of pathological slide images.

### Disclosure of Invention

### Technical Problem

Provided are a method and device for analyzing a pathological slide image. Provided is also a computer-readable recording medium having recorded thereon a program for causing a computer to execute the method. The technical problems to be solved are not limited to the technical problems as described above, and other technical problems may be present.

### Solution to Problem

A computing device according to an aspect includes: at least one memory; and at least one processor, wherein the processor is configured to divide a pathological slide image into a plurality of patches, determine whether or not at least one artifact is present in at least one of the plurality of patches, and perform an additional task on the basis of a result of the determination.

A method of analyzing a pathological slide image according to another aspect includes: dividing a pathological slide image into a plurality of patches; determining whether or not at least one artifact is present in at least one of the plurality of patches; and performing an additional task on the basis of a result of the determination.

A computer-readable recording medium according to another aspect includes a recording medium having recorded thereon a program for causing a computer to execute the method described above.

### Advantageous Effects of Invention

An area in which a subject is expressed, rather than a background area within a pathological slide image, may be identified, and analysis may be performed within the identified area. In addition, the pathological slide image may be analyzed by a certain area, and thus, medical prediction may be performed even with an image including an artifact.

### Brief Description of Drawings

FIG. 1 is a view illustrating an example of a system for analyzing a pathological slide image, according to an embodiment.
FIG. 2 is a block diagram of a system and a network for providing, processing, and reviewing slide images of tissue specimens by using a machine learning model, according to an embodiment.
FIG. 3A is a block diagram illustrating an example of a user terminal according to an embodiment.
FIG. 3B is a block diagram illustrating an example of a server according to an embodiment.
FIG. 4 is a flowchart illustrating an example of a method of analyzing a pathological slide image, according to an embodiment.
FIG. 5 is a view illustrating a patch according to an embodiment.
FIG. 6 is a view illustrating an example of a patch in which the presence or absence of an artifact is determined, according to an embodiment.
FIG. 7 is a flowchart illustrating an example in which a processor performs an additional task, according to an embodiment.
FIG. 8 is a view illustrating an example of updating a result of a determination on the basis of a user input, according to an embodiment.
FIG. 9 is a view illustrating an example in which a result of analyzing a pathological slide image is output, according to an embodiment.
FIG. 10 is a flowchart illustrating another example in which a processor performs an additional task, according to an embodiment.
FIGS. 11A and 11B are views illustrating an example in which a processor determines a type of an additional task according to a ratio occupied by a first group, according to an embodiment.
FIGS. 12A to 13B are views illustrating examples in which a processor determines a type of an additional task according to a distribution of a first group, according to an embodiment.
FIG. 14 is a flowchart illustrating another example in which a processor performs an additional task, according to an embodiment.
FIG. 15 is a view illustrating an example in which a method of analyzing a pathological slide image is performed by at least one machine learning model, according to an embodiment.

### Best Mode for Carrying out the Invention

A computing device according to an aspect includes: at least one memory; and at least one processor, wherein the processor is configured to divide a pathological slide image into a plurality of patches, determine whether or not at least one artifact is present in at least one of the plurality of patches, and perform an additional task on the basis of a result of the determination.

### Mode for the Invention

Terms used in embodiments are selected as currently widely used general terms as possible, which may vary depending on intentions or precedents of those skilled in the art, emergence of new technologies, and the like. In addition, in certain cases, there are also terms arbitrarily selected by the applicant, and in this case, the meaning thereof will be defined in detail in the description. Therefore, the terms used herein should be defined based on the meanings of the terms and the details throughout the description, rather than the simple names of the terms.

Throughout the description, when a part includes a certain element, it means that other elements may be further included, rather than excluding the other elements, unless otherwise stated. In addition, the term, such as "~ unit" or "~ module" described herein, refers to a unit that processes at least one function or operation, which may be implemented as hardware or software, or a combination of hardware and software.

Also, although the terms, "first", "second", etc. may be used herein to describe various components, these components should not be limited by the terms. The terms may be only used to distinguish one component from another component.

According to an embodiment, "a pathological slide image" may refer to an image obtained by capturing a pathological slide that is fixed and stained via a series of chemical treatment processes for a tissue or the like removed from a human body. In addition, the pathological slide image may refer to a whole slide image (WSI) including a high-resolution image of a whole slide, and may also refer to a portion of the whole slide image, for example, one or more patches. For example, the pathological slide image may refer to a digital image captured or scanned via a scanning apparatus (e.g., a digital scanner or the like), and may include information regarding a particular protein, cell, tissue and/or structure within a human body. In addition, the pathological slide image may include one or more patches, and histological information may be applied (e.g., tagged) to the one or more patches via an annotation task.

According to an embodiment, "medical information" may refer to any medically meaningful information that may be extracted from a medical image, and may include, for example, an area, location, and size of a particular tissue (e.g., a cancer tissue, a cancer stromal tissue, or the like) and/or a particular cell (e.g., a tumor cell, a lymphoid cell, a macrophage cell, an endothelial cell, a fibroblast cell, or the like) within a medical image, diagnostic information regarding cancer, information associated with the possibility of developing cancer of a subject, and/or a medical conclusion associated with cancer treatment, but is not limited thereto. In addition, the medical information may include not only a quantified numerical value that may be obtained from a medical image, but also information obtained by visualizing the numerical value, predictive information according to the numerical value, image information, statistical information, and the like. Medical information generated as described above may be provided to a user terminal or displayed by being output or transmitted to a display device.

Hereinafter, embodiments are described in detail with reference to the accompanying drawings. However, the embodiments may be implemented in several different forms and are not limited to examples described herein.

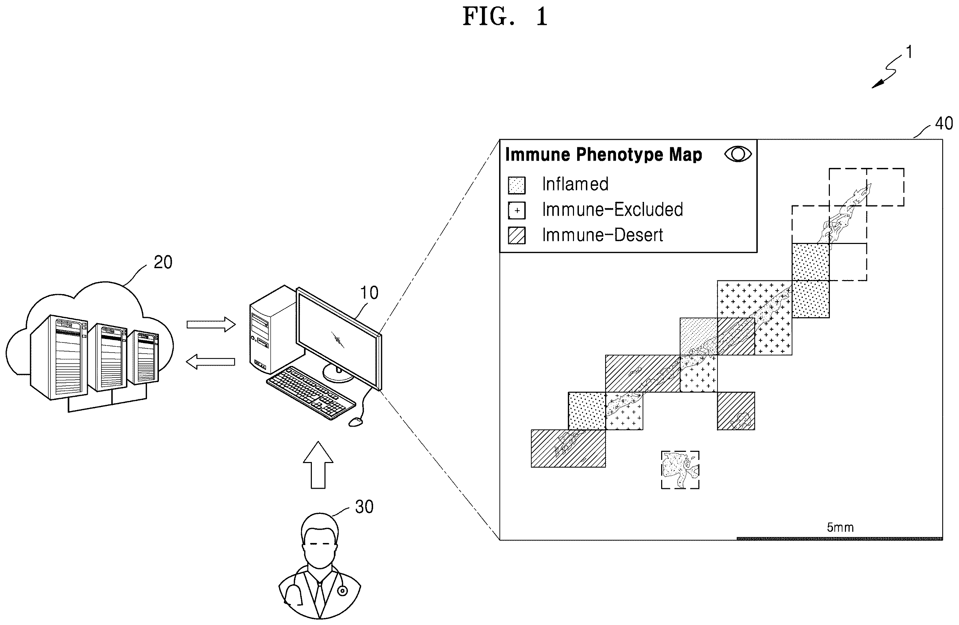

FIG. 1 is a view illustrating an example of a system for analyzing a pathological slide image, according to an embodiment.

Referring to FIG. 1, a system 1 includes a user terminal 10 and a server 20. For example, the user terminal 10 and the server 20 may be connected to each other by a wired or wireless communication method to transmit and/or receive data (e.g., image data or the like) to and/or from each other.

For convenience of description, although FIG. 1 illustrates that the system 1 includes the user terminal 10 and the server 20, the present disclosure is not limited thereto. For example, other external devices (not shown) may be included in the system 1, and operations of the user terminal 10 and the server 20 to be described below may be implemented by a single device (e.g., the user terminal 10 or the server 20) or more devices.

The user terminal 10 may be a computing device that is provided with a display device and a device (e.g., a keyboard, a mouse, or the like) for receiving a user input, and includes a memory and a processor. For example, the user terminal 10 may correspond to a notebook PC, a desktop PC, a laptop, a tablet computer, a smartphone, or the like, but is not limited thereto.

The server 20 may be an apparatus that communicates with an external device (not shown) including the user terminal 10. As an example, the server 20 may be an apparatus that stores various types of data including a pathological slide image, a bitmap image corresponding to the pathological slide image, information generated by analysis of the pathological slide image (e.g., information regarding at least one tissue and cell represented in the pathological slide image, at least one piece of biomarker expression information, or the like), and information regarding a machine learning model used for analysis of the pathological slide image. Alternatively, the server 20 may be a computing apparatus including a memory and a processor, and having an operation capability. In the case where the server 20 is a computing apparatus, the server 20 may perform at least some of operations of the user terminal 10 described below with reference to FIGS. 1 to 15. For example, the server 20 may also be a cloud server, but is not limited thereto.

The user terminal 10 outputs an image 40 expressing information generated via analysis of a pathological slide image and/or a pathological slide. For example, various types of information regarding at least one tissue and cell expressed in the pathological slide image may be expressed in the image 40. In addition, expression information of a biomarker may be expressed in the image 40. In addition, the image 40 may be a report including medical information regarding at least a partial area included in the pathological slide image. Also, the image 40 may be a report including a result of evaluation of the pathological slide image.

The pathological slide image may refer to an image obtained by capturing a pathological slide that is fixed and stained through a series of chemical treatment processes to observe a tissue or the like removed from a human body under a microscope. As an example, the pathological slide image may refer to a whole slide image including a high-resolution image for a whole slide. As another example, the pathological slide image may refer to a portion of the high-resolution whole slide image.

Meanwhile, the pathological slide image may refer to a patch area divided into patch units from the whole slide image. For example, a patch may have a size of a certain area. Alternatively, the patch may refer to an area including each of objects included within the whole slide.

In addition, the pathological slide image may refer to a digital image captured by using a microscope and may include information regarding a cell, a tissue, and/or a structure within a human body.

By analyzing the pathological slide image, biological factors (e.g., a cancer cell, an immune cell, a cancer area, and the like) expressed in the pathological slide image may be identified. The biological factors may be used for a histological diagnosis of a disease, prediction of a disease prognosis, a determination of treatment direction for a disease, or the like.

The quality of the pathological slide image may be deteriorated by various factors. For example, the quality of the pathological slide image may deteriorate due to an issue that occurs in a process of producing a slide or in a process after the slide is produced. In addition, the quality of the pathological slide image may deteriorate due to an issue that occurs in a process of scanning the slide with a scanner.

A n artifact distorts an original image, and thus, accurate information is not easily identified from a pathological slide image in which an artifact is present. In general, a user 30 often identifies needed information from a readable area excluding, from the pathological slide image, an area in which reading is not easy or available due to an artifact. Alternatively, in the case where an area in which reading is not easy or available occupies more than a certain portion of the pathological slide image, the user 30 re-produces the slide or scans the slide again to perform reading. Accordingly, an issue may occur in which the result of reading the pathological slide image is inaccurate or the time or cost taken for reading increases.

The user terminal 10 according to an embodiment divides the pathological slide image into a plurality of patches, and determines whether or not an artifact is present in at least one of the patches. In addition, the user terminal 10 performs an additional task on the basis of the result of the determination.

In other words, the user terminal 10 not only identifies a patch including at least one artifact (hereinafter, referred to as a noise patch) in the pathological slide image, but also performs various additional tasks on the pathological slide image including the noise patch. For example, the user terminal 10 may output, on the image 40, the result of analysis of the pathological slide image including the noise patch. Alternatively, the user 30 may also modify information that is output on the image 40. Alternatively, the user terminal 10 may stop analysis of the pathological slide image or output, on the image 40, the result of analysis of the pathological slide image excluding the noise patch. Therefore, the utilization of or the degree of analysis of the pathological slide image including the noise patch may be improved.

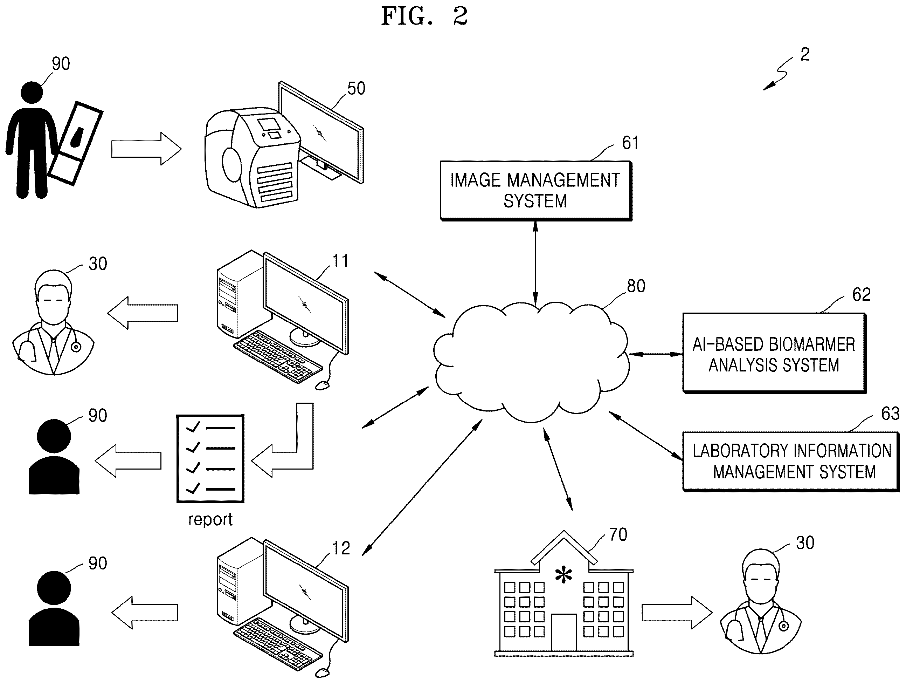

Hereinafter, an example in which the user terminal 10 performs at least one additional task on the basis of the result of determining whether or not an artifact is present in patches is described with reference to FIGS. 2 to 15.

Meanwhile, for convenience of description, the user terminal 10 is described as performing operations according to an embodiment throughout the description, but is not limited thereto. For example, at least some of the operations performed by the user terminal 10 may also be performed by the server 20.

In other words, at least some of operations of the user terminal 10 described with reference to FIGS. 1 to 15 may be performed by the server 20. For example, the server 20 may divide a pathological slide image into a plurality of patches. In addition, the server 20 may determine whether or not an artifact is present in at least one of the plurality of patches. In addition, the server 20 may perform an additional task according to the result of the determination. Also, the server 20 may transmit, to the user terminal 10, the result of the determination and/or the result of performing the additional task. However, an operation of the server 20 is not limited to the above description.

FIG. 2 is a block diagram of a system and a network for providing, processing, and reviewing slide images of tissue specimens by using a machine learning model, according to an embodiment.

Referring to FIG. 2, a system 2 includes user terminals 11 and 12, a scanner 50, an image management system 61, an Al-based biomarker analysis system 62, a laboratory information management system 63, and a hospital or laboratory server 70. In addition, the components (11, 12, 50, 61, 62, 63, 70) included in the system 2 may be connected to one another through a network 80. For example, the network 80 may be a network through which the components (11, 12, 50, 61, 62, 63, and 70) may be connected to one another by using a wired or wireless communication method. For example, the system 2 illustrated in FIG. 2 may include a network that may be connected to servers in hospitals, research rooms, laboratories, and the like, and/or user terminals of doctors or researchers.

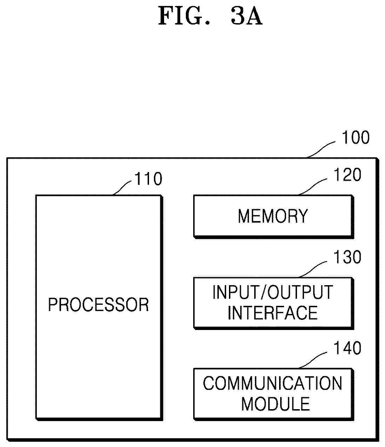

According to various embodiments of the present disclosure, a method described below with reference to FIGS. 3A to 15 may be performed by the user terminals 11 and 12, the image management system 61, the Al-based biomarker analysis system 62, the laboratory information management system 63, and/or the hospital or laboratory server 70.

The scanner 50 may acquire a digitized image from a tissue sample slide generated by using a tissue sample of a subject 90. For example, the scanner 50, the user terminals 11 and 12, the image management system 61, the Al-based biomarker analysis system 62, the laboratory information management system 63, and/or the hospital or laboratory server 70 may be connected to the network 80, such as the Internet, via one or more computers, servers, and/or mobile devices, respectively, or may communicate with the user 30 and/or the subject 90 via one or more computers, and/or mobile devices.

The user terminals 11 and 12, the image management system 61, the Al-based biomarker analysis system 62, the laboratory information management system 63, and/or the hospital or laboratory server 70 may generate or otherwise acquire, from another apparatus, one or more tissue samples of the subject 90, tissue sample slides, digitized images of the tissue sample slides, or any combination thereof. In addition, the user terminals 11 and 12, the image management system 61, the Al-based biomarker analysis system 62, and the laboratory information management system 63 may acquire any combination of subject-specific information, such as age, medical history, cancer treatment history, family history, and past biopsy records of the subject 90, or disease information of the subject 90.

The scanner 50, the user terminals 11 and 12, the image management system 61, the laboratory information management system 63, and/or the hospital or laboratory server 70 may transmit digitized slide images and/or subject-specific information to the Al-based biomarker analysis system 62 via the network 80. The Al-based biomarker analysis system 62 may include one or more storage devices (not shown) for storing received images and data. In addition, the Al-based biomarker analysis system 62 may include a machine learning model repository that stores a machine learning model trained to process the received images and data. For example, the Al-based biomarker analysis system 62 may include a machine learning model that is learned and trained to predict, from a pathological slide image of the subject 90, at least one of information regarding at least one cell, information regarding at least one area, information related to a biomarker, medical diagnostic information, and/or medical treatment information.

The scanner 50, the user terminals 11 and 12, the Al-based biomarker analysis system 62, the laboratory information management system 63, and/or the hospital or laboratory server 70 may transmit a digitized slide image, subject-specific information, and/or a result of analysis of the digitized slide image to the image management system 61 via the network 80. The image management system 61 may include a repository for storing a received image and a repository for storing a result of analysis.

In addition, according to various embodiments of the present disclosure, a machine learning model, which is learned and trained to predict, from a slide image of the subject 90, at least one of information regarding at least one cell, information regarding at least one area, information related to a biomarker, medical diagnostic information, and/or medical treatment information, may be stored in the user terminals 11 and 12 and/or the image management system 61 and operate.

According to various embodiments of the present disclosure, a pathological slide image analysis method, a subject information processing method, a subject group selection method, a clinical trial design method, a biomarker expression information generation method, and/or a method of setting a reference value for a particular biomarker may be performed not only by the Al-based biomarker analysis system 62, but also by the user terminals 11 and 12, the image management system 61, the laboratory information management system 63, and/or the hospital or laboratory server 70.

FIG. 3A is a block diagram illustrating an example of a user terminal according to an embodiment.

Referring to FIG. 3A, a user terminal 100 includes a processor 110, a memory 120, an input/output interface 130, and a communication module 140. For convenience of description, FIG. 3A illustrates only components related to the present disclosure. Accordingly, the user terminal 100 may further include other general-purpose components, in addition to the components shown in FIG. 3A. In addition, it is obvious to those skilled in the art related to the present disclosure that the processor 110, the memory 120, the input/output interface 130, and the communication module 140 shown in FIG. 3A may also be implemented as independent devices.

In addition, an operation of the user terminal 100 may be performed by the user terminals 11 and 12, the image management system 61, the Al-based biomarker analysis system 62, the laboratory information management system 63, and/or the hospital or laboratory server 70 of FIG. 2.

The processor 110 may process commands of a computer program by performing basic arithmetic, logic, and input/output operations. Here, the commands may be provided from the memory 120 or an external apparatus (e.g., the server 20 or the like). In addition, the processor 110 may control overall operations of other components included in the user terminal 100.

The processor 110 divides a pathological slide image into a plurality of patches. In addition, the processor 110 determines whether or not at least one artifact is present in at least one of the plurality of patches. For example, the processor 110 may determine whether or not an artifact is present only in at least one patch needing to be analyzed, from among the plurality of patches.

The processor 110 performs an additional task on the basis of the result of the determination.

As an example, the processor 110 may output information regarding a plurality of patches on which the result of the determination is reflected, and update the result of the determination on the basis of a user input according to identification of the output information. For example, the processor 110 may output information regarding patches according to at least one of a first type (a type that distinguishes a plurality of patches according to whether or not an artifact is present therein) and a second type (a type that distinguishes a plurality of patches into a patch that needs to be analyzed, a patch that does not need to be analyzed, or a noise patch (i.e., a patch including at least one artifact).

As another example, the processor 110 may determine a type of an additional task according to a relationship between a first group of patches in which at least one artifact is present and a second group of patches in which at least one artifact is not present. In addition, the processor 110 may automatically perform a determined additional task or output information regarding a determined additional task. Here, the relationship may refer to at least one of a ratio occupied by the first group in the sum of the first group and the second group, a distribution of the first group in the sum of the first group and the second group, and a location of the first group in the sum of the first group and the second group.

As another example, the processor 110 may determine the type of the additional task according to a relationship between whether or not at least one noise patch is removed and quality of analysis of the pathological slide image.

For example, the additional task may include at least one of a first task of stopping analysis of the pathological slide image, a second task of analyzing the rest excluding at least one noise patch from the pathological slide image, and a third task of analyzing the whole pathological slide image.

The processor 110 may be implemented as an array of a plurality of logic gates, or may be implemented as a combination of a general-purpose microprocessor and a memory that stores a program executable by the microprocessor. For example, the processor 110 may include a general-purpose processor, a central processing unit (CPU), a microprocessor, a digital signal processor (DSP), a controller, a microcontroller, a state machine, or the like. In some environments, the processor 110 may include an application-specific integrated circuit (ASIC), a programmable logic device (PLD), a field programmable gate array (FPGA), or the like. For example, processor 110 may refer to a combination of processing devices, such as a combination of a digital signal processor (DSP) and a microprocessor, a combination of a plurality of microprocessors, a combination of one or more microprocessors combined with a digital signal processor (DSP) core, or a combination of any other such components.

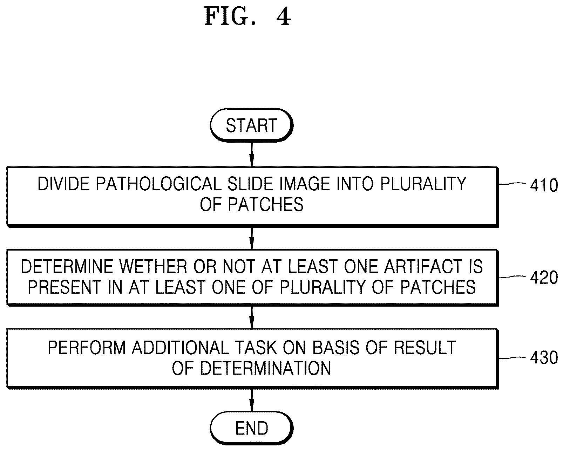

The memory 120 may include any non-transitory computer-readable recording medium. As an example, the memory 120 may include a non-volatile (permanent) mass storage device, such as random access memory (RAM), read only memory (ROM), a disk drive, a solid state drive (SSD), or flash memory. As another example, the non-volatile mass storage device, such as ROM, an SSD, flash memory, or a disk drive, may be a separate permanent storage device distinguished from a memory. Also, the memory 210 may store an operating system (OS) and at least one program code (e.g., a code for the processor 110 to perform an operation described below with reference to FIGS. 4 to 15).

Software components described above may be loaded from a computer-readable recording medium separate from the memory 120. Such a separate computer-readable recording medium may be a recording medium that may be directly connected to the user terminal 100, and may include, for example, a computer-readable recording medium, such as a floppy drive, a disk, a tape, a DVD/CD-ROM drive, or a memory card. Alternatively, the software components may also be loaded into the memory 120 via the communication module 140 rather than a computer-readable recording medium. For example, at least one program may be loaded into the memory 120 on the basis of a computer program (e.g., a computer program for the processor 110 to perform an operation described below with reference to FIGS. 4 to 15, or the like) installed by files provided via the communication module 140 by developers or a file distribution system that distributes installation files of applications.

The input/output interface 130 may be a means for an interface with a device (e.g., a keyboard, a mouse, or the like) for an input or an output, which may be connected to the user terminal 100 or included in the user terminal 100. Although FIG. 3A illustrates that the input/output interface 130 is an element configured separately from the processor 110, the input/output interface 130 is not limited thereto, and may also be configured to be included in the processor 110.

The communication module 140 may provide a component or function for the server 20 and the user terminal 100 to communicate with each other via a network. In addition, the communication module 140 may provide a component or function for the user terminal 100 to communicate with another external device. For example, a control signal, a command, data, and the like, which are provided under control of the processor 110, may be transmitted to the server 20 and/or an external device through the communication module 140 and the network.

Meanwhile, although not shown in FIG. 3A, the user terminal 100 may further include a display device. Alternatively, the user terminal 100 may be connected to an independent display device via a wired or wireless communication method to transmit and/or receive data to and/or from each other. For example, a pathological slide image, analysis information of the pathological slide image, prediction information of treatment response, and the like may be provided to the user 30 via the display device.

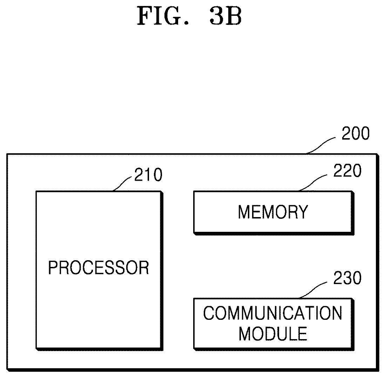

FIG. 3B is a block diagram illustrating an example of a server according to an embodiment.

Referring to FIG. 3B, a server 20 includes a processor 210, a memory 220, and a communication module 230. For convenience of description, FIG. 3B illustrates only components related to the present disclosure. Accordingly, the server 200 may further include other general-purpose components, in addition to the components shown in FIG. 3B. In addition, it is obvious to those skilled in the art related to the present disclosure that the processor 210, the memory 220, and the communication module 230 shown in FIG. 3B may also be implemented as independent apparatuses.

The processor 210 may acquire a pathological slide image from at least one of the internal memory 220, an external memory (not shown), the user terminal 100, or an external apparatus. The processor 210 may divide the pathological slide image into a plurality of patches, determine whether or not at least one artifact is present in at least one of the plurality of patches, or perform an additional task according to the result of the determination.

In other words, at least one of the operations of the processor 110 described above with reference to FIG. 3A may be performed by the processor 210. In this case, the user terminal 100 may output, through a display device, information transmitted from the server 200.

Meanwhile, an implementation example of the processor 210 is the same as the implementation example of the processor 110 described above with reference to FIG. 3A, and thus, a detailed description thereof is omitted.

Various types of data such as a pathological slide image, and data generated according to the operation of the processor 210 may be stored in the memory 220. In addition, the memory 220 may store an operating system (OS) and at least one program (e.g., a program needed for the processor 210 to operate).

Meanwhile, an implementation example of the memory 220 is the same as the implementation example of the memory 120 described above with reference to FIG. 3A, and thus, a detailed description thereof is omitted.

The communication module 230 may provide a component or function for the server 200 and the user terminal 100 to communicate with each other through a network. Also, the communication module 230 may provide a component or function for the server 200 to communicate with another external device. For example, a control signal, a command, data, and the like provided under control of the processor 210 may be transmitted to the user terminal 100 and/or an external device through the communication module 230 and the network.

FIG. 4 is a flowchart illustrating an example of a method of analyzing a pathological slide image, according to an embodiment.

Referring to FIG. 4, the method of analyzing the pathological slide image includes operations processed in time series by the user terminal 10 or 100 or the processor 110 illustrated in FIG. 1 or 3A. Therefore, even in the case where the above description of the user terminal 10 or 100 or the processor 110 illustrated in FIG. 1 or 3A is omitted, the above description may also be applied to the method of analyzing the pathological slide image, illustrated in FIG. 4.

In addition, as described above with reference to FIGS. 1 to 3B, at least one of the operations in the flowchart illustrated in FIG. 4 may be processed by the server 20 or 200, or the processor 210.

In operation 410, the processor 110 divides a pathological slide image into a plurality of patches.

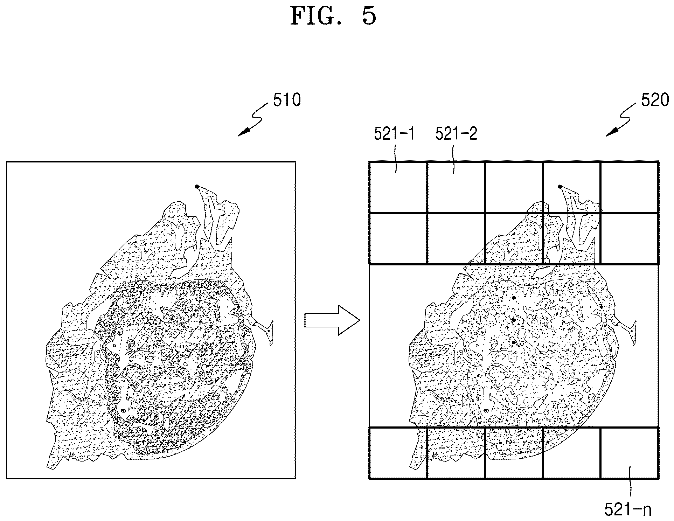

For example, the processor 110 may divide the pathological slide image into at least two patches, and the respective patches may have the same or different sizes. For example, from among the pathological slide image, the processor 110 may divide an area, which needs to be analyzed, into a larger number of patches (e.g., an area in which a subject is displayed, an area of interest, or the like), and a background area into a smaller number of patches. In addition, the processor 110 may acquire patches so that areas of the patches overlap each other within the pathological slide image. Hereinafter, an example of a patch is described with reference to FIG. 5.

FIG. 5 is a view illustrating a patch according to an embodiment.

Referring to FIG. 5, the processor 110 may generate a plurality of patches 521-1, 521-2, ..., and 521-n by dividing a pathological slide image 510. In other words, the pathological slide image 510 may be a combination 520 of a plurality of patches.

FIG. 5 illustrates an example in which the patches 521-1, 521-2, ..., and 521-n are all divided into the same sizes. However, the sizes of the patches 521-1, 521-2, ..., and 521-n) are not limited thereto. For example, from among the pathological slide image, the processor 110 may divide an area, which needs to be analyzed (e.g., an area in which a subject (e.g., a cell, a tissue, a structure, or the like) is displayed, an area of interest, or the like), into small-sized patches and an area, which does not need to be analyzed (e.g., a background area or the like), into large-sized patches. Here, a size of a patch may be preset, or the preset size may be adjusted by the user 30.

Referring back to FIG. 4, in operation 420, the processor 110 determines whether or not at least one artifact is present in at least one of the plurality of patches.

For example, the processor 110 may determine whether or not an artifact is present within a patch by using a predetermined image processing technique or detect areas corresponding to the artifact from the patch by using a machine learning model. Here, the machine learning model may be pre-trained by using noise patches acquired from the pathological slide image including an artifact or noise patches on which intentional image processing is performed to include an artifact. For example, noise patches, which are generated through image processing, such as applying a blur kernel to a patch, applying a color conversion function, inserting a particular figure, generating an effect of a tissue being folded by overlapping a partial area of a patch with another area, or tilting a patch, may be used to train a machine learning model.

An artifact is a concept including various factors that may affect reading of the pathological slide image. In other words, an artifact includes not only foreign substances expressed in an image, but also various factors that lower the quality of the image. For example, an artifact may be generated by the quality of a slide, or may be generated in a process of converting the slide into an image.

As an example, in a process of producing a slide or in a process after producing the slide, an artifact that deteriorates an image quality of the slide may be generated. In the process of producing the slide, a portion of a tissue may be physically pressed or torn in a process of collecting or treating the tissue from the subject 90. Alternatively, during a process of staining the slide, a reagent may over-stain or under-stain a particular area and thus exhibit abnormal color development. Alternatively, foreign substances or air bubble may enter between a cover glass and a specimen, or ink remaining after directly painting a tissue with a pen to check an orientation of a tissue may be included. In the process after the slide is produced, some or all of a specimen on the slide may be physically pressed or torn due to an impact received during a process of transporting the slide. Alternatively, in a process of reading the slide, the user (30) (e.g., a pathologist) may paint the cover glass with the pen for the purpose of displaying a particular area, and thus, ink may be left on the cover glass or the cover glass may be stained with foreign substances or broken.

As another example, an artifact may be generated even in a process of scanning the slide with the scanner 50, and thus, the quality of a final pathological slide image may be lowered. For example, due to irregularities in a tissue included in the slide or issues with a scan lens, a tissue area, which is not clearly scanned or is out of focus, may be included in the image. Alternatively, due to an error in a program or an error in a lamp during the scanning process, at least a portion of the pathological slide image may be scanned excessively dark or bright. Alternatively, due to slight vibrations during the process of scanning the slide, a misalignment between continuously scanned images may occur.

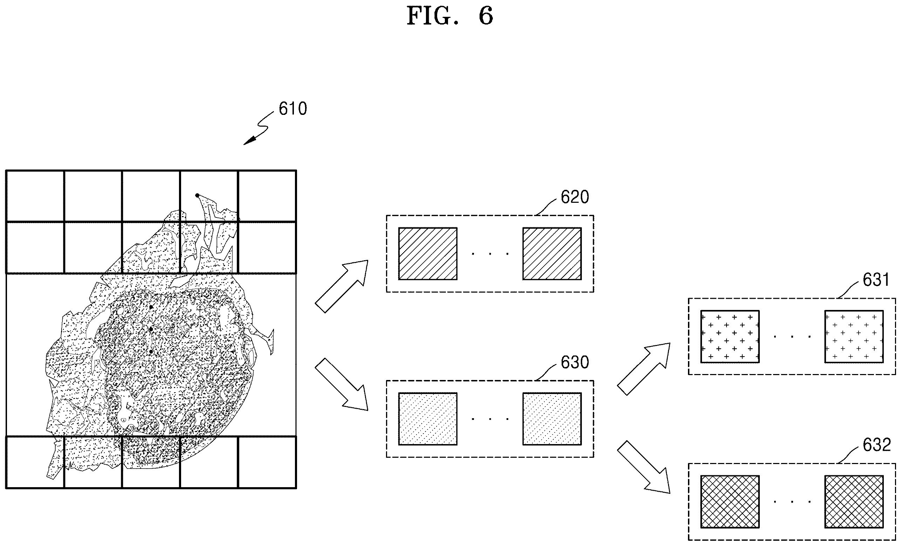

The processor 110 may determine whether or not an artifact is present only in at least one patch needing to be analyzed, from among a plurality of patches. Hereinafter, an example in which the processor 110 selects, from among a plurality of patches, a patch which is determined whether or not an artifact is present therein is described with reference to FIG. 6.

FIG. 6 is a view illustrating an example of a patch which is determined whether or not an artifact is present therein, according to an embodiment.

FIG. 6 illustrates an example in which a pathological slide image 610 is divided into a plurality of patches. The processor 110 may select patches 632 which are determined whether or not an artifact is present therein, from among patches 620, 630, 631, and 632 constituting the pathological slide image 610.

For example, the processor 110 may analyze the patches 620, 630, 631, and 632 and classify the same into patches 620 including only a background area and patches 630 including a subject. For example, the processor 110 may analyze the pathological slide image 610 by using a predetermined image processing technique or detect areas corresponding to the subject from the pathological slide image 610 by using a machine learning model. Accordingly, the processor 110 may distinguish, in the pathological slide image 610, between the patches 620 including only the background area and the patches 630 including the subject.

In addition, the processor 110 may classify the patches 630 into the patches 631 and the patches 632 according to a preset criterion. For example, the processor 110 may select, from among the patches 630, the patches 631 which include the subject at a ratio lower than or equal to a certain ratio or does not include an area of interest. Here, a ratio at which a subject is included in a patch, and an area of interest may be preset in the user terminal 100, or may be set or adjusted by the user 30. Accordingly, the processor 110 may select the patches 632 as targets which are determined whether or not an artifact is present therein.

Meanwhile, according to the above description, the processor 110 selects, from the pathological slide image 610, only the patches 632 as the targets which are determined whether or not an artifact is present therein, but is not limited thereto. In some cases, the processor 110 may also select the patches 630 as targets which are determined whether or not an artifact is present therein. In addition, in some cases, the processor 110 may select the patches 620 and 630 as targets which are determined whether or not an artifact is present therein.

Referring back to FIG. 4, in operation 430, the processor 110 performs an additional task on the basis of the result of the determination.

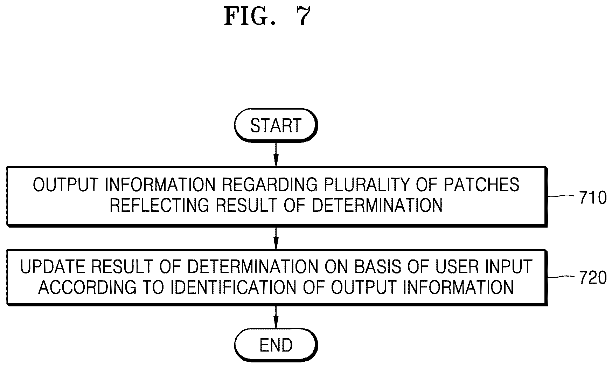

As an example, the processor 110 may output information regarding a plurality of patches on which the result of the determination in operation 420 is reflected. In addition, the processor 110 may update the result of the determination on the basis of a user input. Here, the user input may be received as the user 30 identifies information regarding the plurality of patches, which is previously output. An example in which the processor 110 performs an additional task is described below with reference to FIGS. 7 to 9.

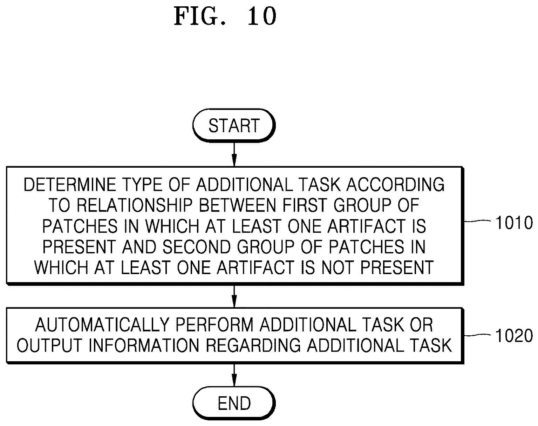

As another example, the processor 110 may determine a type of the additional task according to a relationship between a first group of patches in which at least one artifact is present and a second group of patches in which at least one artifact is not present. Here, the first group refers to a group that includes noise patches, and the second group refers to a group that does not include noise patches. In addition, the processor 110 may automatically perform the additional task or output information regarding the additional task. Another example in which the processor 110 performs an additional task is described below with reference to FIGS. 10 to 13B.

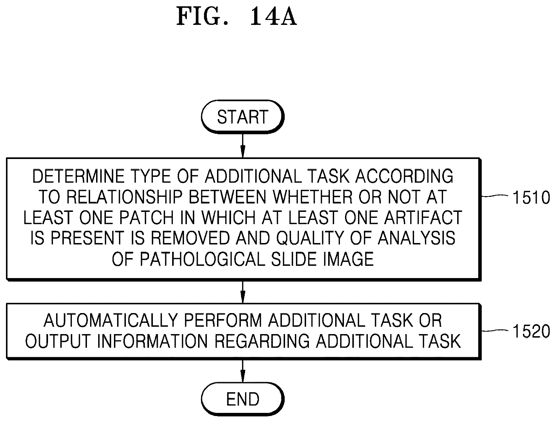

As another example, the processor 110 may determine the type of the additional task according to a relationship between whether or not at least one noise patch (i.e., a patch in which at least one artifact is present) is removed and quality of analysis of the pathological slide image. In addition, the processor 110 may automatically perform the additional task or output information regarding the additional task. Another example in which the processor 110 performs the additional task is described below with reference to FIG. 14.

For example, the additional task may include at least one of a first task of stopping analysis of the pathological slide image, a second task of analyzing the rest excluding at least one noise patch from the pathological slide image, and a third task of analyzing the whole pathological slide image.

FIG. 7 is a flowchart illustrating an example in which a processor performs an additional task, according to an embodiment.

In operation 710, the processor 110 outputs information regarding a plurality of patches on which the result of the determination in operation 420 is reflected.

For example, the processor 110 may output the information regarding the patches according to a first type that distinguishes the plurality of patches according to whether or not an artifact is present and a second type that distinguishes the plurality of patches into a patch that needs to be analyzed, a patch that does not need to be analyzed, or a patch (i.e., a noise patch) that includes an artifact.

In the case of the first type, the processor 110 may output a patch including an artifact (i.e., a noise patch) in distinction from another patch. Here, the processor 110 may overlay even information regarding an identified artifact on patches.

In the case of the second type, the processor 110 may output a patch that needs to be analyzed, a patch that does not need to be analyzed, and a noise patch separately from one another. In this case, the processor 110 may output together analysis information (e.g., biomarker information) regarding the patch that does not need to be analyzed. Here, the processor 110 may overlay even the identified artifact on the patches.

In operation 720, the processor 110 updates the result of the determination on the basis of a user input according to identification of the output information.

According to an operation of the processor 110, a noise patch may not be accurately detected from a pathological slide image. Accordingly, the processor 110 may update the result of the detection of the noise patch, on the basis of a user input related to an addition/change/deletion of the noise patch.

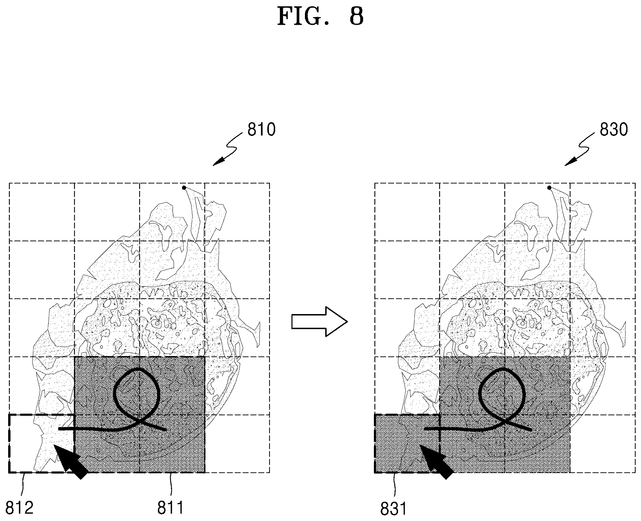

FIG. 8 is a view illustrating an example of updating a result of determination on the basis of a user input, according to an embodiment.

FIG. 8 illustrates a pathological slide image 810 before the result of detecting a noise patch is updated and a pathological slide image 830 after the result is updated. The example illustrated in FIG. 8 is an example in which information regarding patches is output according to the first type described above.

The processor 110 may output information regarding patches of the pathological slide image 810. For example, noise patches 811 detected as operation 420 is performed may be output in distinction from other patches of the pathological slide image 810.

Referring to a location of an artifact in the pathological slide image 810, the artifact is also included in a patch 812. Here, it is assumed that the processor 110 fails to identify that an artifact is present in the patch 812.

After the information regarding the patches of the pathological slide image 810 is output, the user 30 may input a command to add even the patch 812 to the noise patches 811. Here, the processor 110 may add even a patch 831 to the noise patches 811 according to a user input, and accordingly, an updated result 830 may be output.

Although FIG. 8 illustrates an example in which the noise patches 812 and 831 are added, the present disclosure is not limited thereto. In the same manner as described above, the user 30 may input a command related to change/deletion of a noise patch, and the processor 110 may update the result of detection of the noise patch, on the basis of the user input.

Meanwhile, although not illustrated in FIG. 8, the processor 110 may also output information regarding a biomarker by analyzing the remaining patches excluding the noise patches 811 and 812.

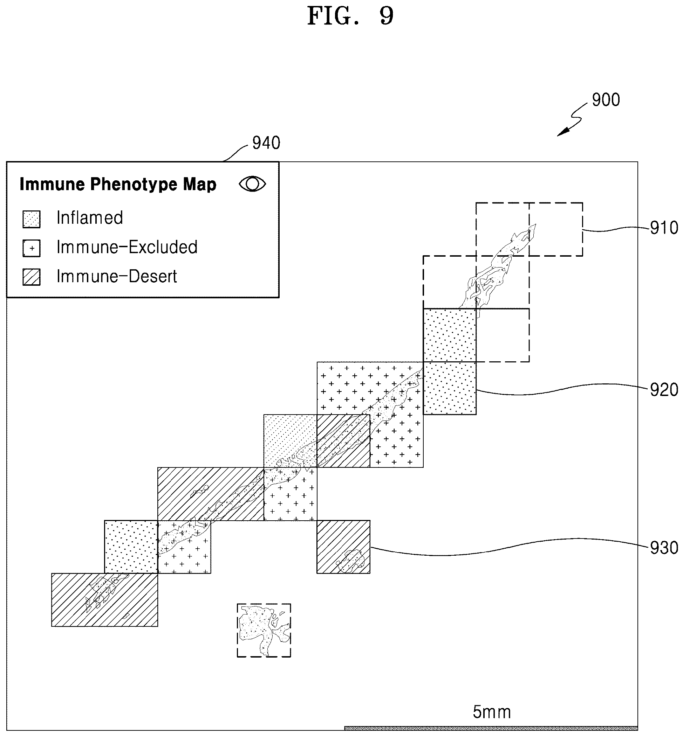

FIG. 9 is a view illustrating an example in which a result of analysis of a pathological slide image is output, according to an embodiment.

FIG. 9 illustrates an example in which information regarding patches is output according to the second type described above. For example, patches 910 may be patches that do not need to be analyzed, patches 920 may be noise patches, and patches 930 may be patches that need to be analyzed. FIG. 9 illustrates that information related to an artifact is not overlaid and displayed on patches, but an artifact may be displayed on patches as described above.

Meanwhile, the processor 110 may additionally output information 940 regarding a biomarker by analyzing the patches 930. For example, assuming that the information 940 regarding the biomarker is an immune phenotype, the processor 110 may output the patches 930 in distinction from one another according to the immune phenotype.

In addition, although not illustrated in FIG. 9, in the case where a noise patch that is not detected by the processor 110 is present, as described above with reference to FIG. 8, information regarding the noise patch may be updated on the basis of a user input.

FIG. 10 is a flowchart illustrating another example in which a processor performs an additional task, according to an embodiment.

In operation 1010, the processor 110 determines a type of an additional task according to a relationship between a first group of patches in which at least one artifact is present and a second group of patches in which at least one artifact is not present.

Here, the first group refers to a group including at least one noise patch, and the second group refers to a group including patches that do not belong to the first group, from among analyzable patches included in a pathological slide image. In other words, the patches included in the first group and the patches included in the second group include the analyzable patches included in the pathological slide image. For example, the analyzable patches may be patches including a subject, and may be the rest excluding patches including only a background area from among the patches constituting the pathological slide image.

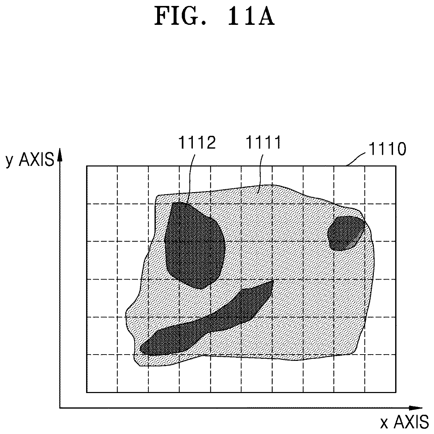

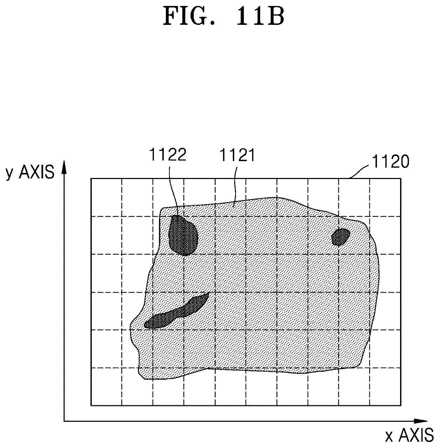

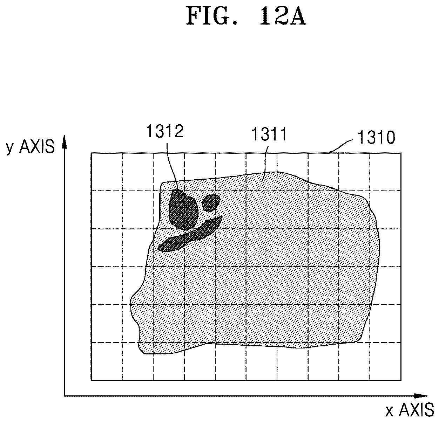

For example, the relationship may include at least one of a ratio occupied by the first group in the sum of the first group and the second group, a distribution of the first group in the sum of the first group and the second group, and a location of the first group in the sum of the first group and the second group. An example in which the processor 110 determines a type of an additional task according to a ratio occupied by a first group is described below with reference to FIGS. 11A and 11B. In addition, an example in which the processor 110 determines a type of an additional task according to a distribution of a first group is described below with reference to FIGS. 12A to 13B.

In operation 1020, the processor 110 automatically performs the additional task or outputs information regarding the additional task.

For example, the additional task may include at least one of a first task of stopping analysis of the pathological slide image, a second task of analyzing the rest excluding at least one noise patch from the pathological slide image, and a third task of analyzing the whole pathological slide image.

In the case of the first task, the processor 110 may determine that the pathological slide image is unanalyzable and recommend re-scanning of an image or re-producing of a slide. Alternatively, the processor 110 may stop analysis of the pathological slide image and guide the user 30 into an additional action (e.g., cleaning or re-staining of a slide, or the like).

In the case of the second task, the processor 110 may perform analysis of the remaining patches excluding noise patches from the pathological slide image. In addition, the processor 110 may output both the result of analysis performed after excluding a noise patch and the result of analysis of the whole pathological slide image from which a noise patch is not excluded. In the case where the two results described above are different from each other, the processor 110 may provide an alarm so that the user 30 may review a corresponding slide in detail. In addition, the processor 110 may also perform analysis after excluding the whole area corresponding to the noise patches from the pathological slide image or excluding only an area identified as an artifact within the noise patches.

In the case of the third task, the processor 110 may derive expression information of a biomarker by analyzing the whole analyzable area including the noise patches. The processor 110 may output together the result of analysis performed after excluding the noise patch and the result of analysis performed without excluding the analyzable noise patch. In this case, in the case where a significant difference (e.g., a result difference that may affect decision-making of the user 30) is present between the two results, the processor 110 may guide the user 30 to the difference.

In the case where the processor 110 outputs information regarding an additional task, the user 30 may determine whether or not to perform the additional task after identifying the information provided by the processor 110.

FIGS. 11A and 11B are views illustrating an example in which a processor determines a type of an additional task according to a ratio occupied by a first group, according to an embodiment.

As a ratio occupied by a noise patch from among analyzable patches of a pathological slide image (i.e., a ratio occupied by the first group) is greater, accuracy of a result of analysis of the pathological slide image may become lower. Accordingly, as the processor 110 determines different types of additional tasks according to a ratio occupied by patches of the first group from among patches included in the first group and the second group, the accuracy of the analysis of the pathological slide image may be increased.

For example, an example in which the processor 110 calculates the ratio occupied by the first group in the sum of the first group and the second group is as follows. First, the processor 110 identifies analyzable patches by excluding patches that include a background and thus are not included in analysis, from among a plurality of patches constituting the pathological slide image. In addition, the processor 110 calculates a ratio occupied by the number of noise patches from among the number of analyzable patches.

Referring to FIGS. 11A and 11B, it is assumed that, in pathological slide images 1110 and 1120, the total number of patches of is 60, and the number of analyzable patches (patches including shaded portions) is 46. From among the same, the noise patches are assumed to be patches including dark shaded portions.

Referring to FIG. 11A, the total number of a first group (patches including dark shaded portions) 1112 and a second group (patches including light shaded portions) 1111 is 46. From among the same, 18 noise patches 1112 are present, and thus, the processor 110 calculates a ratio occupied by the first group 1112 in the sum of the first group 1112 and the second group 1111 as 18/46=0.39.

Referring to FIG. 11B, as in FIG. 11A, the sum of A first group (patches including a dark shaded portion) 1122 and a second group (patches including a light shaded portion) 1121 is a total of 46. From among the same, seven noise patches 1122 are present, and thus, the processor 110 calculates a ratio occupied by the first group 1122 in the sum of the first group 1122 and the second group 1121 as 7/46=0.15.

As described above, in the case where the ratios occupied by the first groups 1112 and 1122 are calculated, the processor 110 determines a type of an additional task on the basis of the calculated ratios. In addition, the processor 110 automatically performs the additional task or outputs information regarding the additional task.

First, examples in which the processor 110 automatically performs an additional task are as follows.

As an example, in the case where a calculated ratio exceeds a first threshold ratio, the processor 110 may perform a first task of stopping analysis of the pathological slide image. In addition, the processor 110 may start a task for a subsequent action (e.g., cleaning or re-staining of a slide or the like).

As another example, in the case where the calculated ratio is less than or equal to the first threshold ratio and exceeds a second threshold ratio, a second task of analyzing the rest excluding a noise patch from the pathological slide image may be performed. In addition, the processor 110 may output the result of analysis. Here, the processor 110 may exclude the whole noise patch from an analysis target or may exclude only an artifact area from the analysis target by segmenting the artifact area included within the noise patch.

As another example, in the case where the calculated ratio is less than or equal to the second threshold ratio, the processor 110 may perform a third task of analyzing the whole pathological slide image. In addition, the processor 110 may output the result of analysis.

Here, the first threshold ratio and the second threshold ratio may be determined in advance, or may be set or adjusted by the user 30.

As another example, the processor 110 may also provide data for the user 30 to determine a subsequent task by outputting together the result of analysis performed after excluding a noise patch from the pathological slide image (i.e., the result of the second task) and the result of analysis performed without excluding the noise patch (i.e., the result of the third task).

Subsequently, examples in which the processor 110 outputs information regarding an additional task are as follows. In the case where the processor 110 outputs the information regarding the additional task, the user 30 may determine a subsequent task and perform the same.

As an example, in the case where the calculated ratio exceeds the first threshold ratio, the processor 110 may notify that the stop of analysis of the pathological slide image and a subsequent action (e.g., cleaning or re-staining of a slide or the like) are needed.

As another example, in the case where the calculated ratio is less than or equal to the first threshold ratio, the processor 110 may output together the result of analysis performed after excluding the noise patch from the pathological slide image and the result of analysis performed without excluding the noise patch. In addition, in the case where a pathological result difference, which may affect decision-making, is present between the two results, the processor 110 may additionally notify the same to the user (e.g., output a warning or the like).

FIGS. 12A to 13B are views illustrating examples in which a processor determines a type of an additional task according to a distribution of a first group, according to an embodiment.

The processor 110 may determine a type of an additional task according to a distribution of a first group in the sum of the first group and a second group. Here, the distribution of the first group in the sum of the first group and the second group may be expressed as the degree of dispersion of noise patches in a pathological slide image.

As the degree of dispersion of noise patches in the pathological slide image is greater, homogeneity and representativeness of an area of interest are lowered. Alternatively, as the noise patches are more concentrated in the pathological slide image, the reliability of the result of analysis of the pathological slide image may be significantly lowered. The processor 110 may perform different subsequent procedures according to the distribution of noise patches (or the degree of dispersion of noise patches). Accordingly, the accuracy of analysis of the pathological slide image may increase.

As an example, an example in which the processor 110 calculates the distribution occupied by the first group in the sum of the first group and the second group is as follows. First, the processor 110 obtains coordinates (e.g., twodimensional coordinates) of each of a plurality of patches constituting the pathological slide image. In addition, the processor 110 counts the number of analyzable patches having corresponding x-coordinate (or corresponding y-coordinate) values along the x-axis (or along the y-axis) and the number of noise patches. In addition, the processor 110 can calculate the degree of disperse in which the noise patches are distributed within the pathological slide image, by calculating a ratio of the number of noise patches (i.e., the number of first group) to the number of analyzable patches (i.e., the sum of the number of patches in the first group and the number of patches in the second group) corresponding to each coordinate value (i.e., counted along the x-axis (i.e., along the y-axis)).

In the case where an area having a high ratio of the number of noise patches to the number of analyzable patches is concentrated along a certain x-axis coordinate value and a certain y-axis coordinate value, the processor 110 may determine that the degree of dispersion in which the noise patches are distributed is low (i.e., the density of the noise patches is high). In contrast, in the case where an area in which the ratio of the number of noise patches to the number of analyzable is low is widely distributed along the x-axis and the y-axis, the processor 110 may determine that the degree of dispersion in which the noise patches are distributed is high (i.e., the density of noise patches is low).

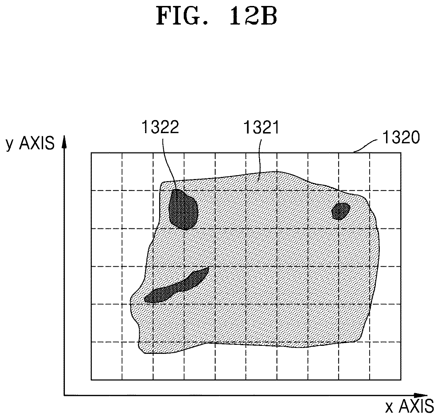

FIGS. 12A and 12B illustrate two examples in which shapes, locations, and areas occupied by analyzable areas (shaded portions) 1311 and 1321 are the same as each other within pathological slide images 1310 and 1320. In each of FIGS. 12A and 12B, three artifacts (dark shaded portions) 1312 or 1322 are located within the pathological slide image 1310 or 1320.

Referring to FIG. 12A, the artifacts 1312 are densely located within the analyzable area 1311. In this case, the processor 110 may determine that an area in which a ratio of the number of noise patches (i.e., the number of patches including the artifacts 1312) to the number of analyzable patches (i.e., the number of patches including the artifacts 1311) is high is concentrated along the x axis and the y axis. Accordingly, the processor 110 may determine that the degree of dispersion of noise patches in the pathological slide image 1310 is relatively low.

Referring to FIG. 12B, the artifacts 1322 are relatively dispersed within the analyzable area 1321. In this case, the processor 110 may determine that an area in which the ratio of the number of noise patches (i.e., the number of patches including the artifacts 1322) to the number of analyzable patches (i.e., the number of patches including the analyzable area 1321) is low is widely distributed along the x axis and the y axis. Accordingly, the processor 110 may determine that the degree of dispersion of the noise patches in the pathological slide image 1320 is relatively high.

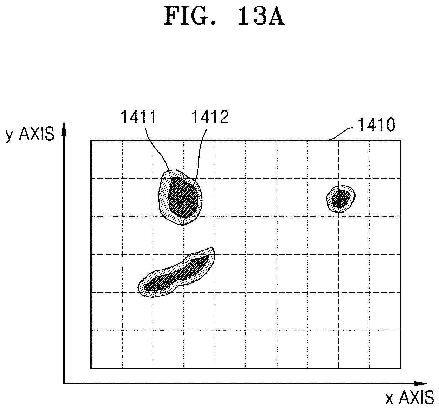

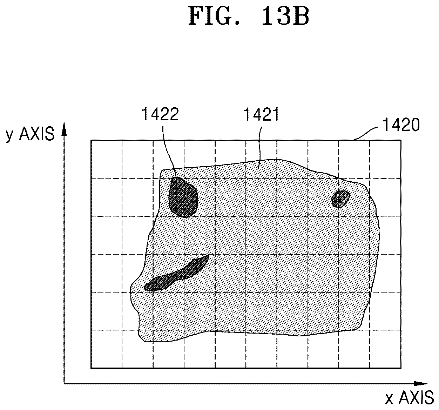

FIGS. 13A and 13B illustrate two cases where shapes, locations, and areas occupied by artifacts (dark shaded portions) 1412 and 1422 are the same as each other within pathological slide images 1410 and 1420. In each of FIGS. 13A and 13B, three artifacts 1412 or 1422 are located far apart within the pathological slide image 1410 or 1420.

Referring to FIG. 13A, within the analyzable area (the shaded portion) 1411, the artifact 1412 occupies most of an area and is densely located. In this case, the processor 110 may determine that an area in which a ratio of the number of noise patches (i.e., the number of patches including the artifacts 1412) to the number of analyzable patches (i.e., the number of patches including the artifacts 1411) is high is concentrated along the x axis and the y axis. Accordingly, the processor 110 may determine that the degree of dispersion of the noise patches within the pathological slide image 1410 is relatively low.

Referring to FIG. 13B, within the analyzable area (the shaded portion) 1421, the artifacts 1422 occupies an excessively partial area and is relatively dispersed. In this case, the processor 110 may determine that an area in which the ratio of the number of noise patches (i.e., the number of patches including the noise area 1422) to the number of analyzable patches (i.e., the number of patches including the analyzable area 1421) is low is widely distributed along the x axis and the y axis. Accordingly, the processor 110 determines that the degree of dispersion of noise patches within the pathological slide image 1420 is relatively high.

As another example, an example in which the processor 110 calculates a distribution occupied by the first group in the sum of the first group and the second group is as follows. First, the processor 110 obtains coordinates of each of patches constituting a pathological slide image. Also, the processor 110 divides the pathological slide image into four quadrants and calculates a ratio of the number of noise patches to the number of analyzable patches, within each quadrant. Accordingly, the processor 110 may determine whether noise patches are evenly distributed in the four quadrants or intensively distributed in at least one quadrant.

As another example, an example in which the processor 110 calculates a distribution occupied by the first group in the sum of the first group and the second group is as follows. First, the processor 110 obtains coordinates of each of the patches constituting the pathological slide image. In addition, the processor 110 designates coordinate values of analyzable patches as a first variable, and designates coordinate values of noise patches as a second variable. In addition, the processor 110 calculates a multi-variable distribution for the first variable and the second variable. Accordingly, the processor 110 may determine whether the degree of dispersion of the noise patches is higher or lower than a distribution of the analyzable patches.

As described above, in the case where the distribution of the first group (or the degree of dispersion of the first group) is identified, the processor 110 determines a type of additional task on the basis of the identified distribution (or the degree of dispersion). In addition, the processor 110 automatically performs the additional task or outputs information regarding the additional task.

First, examples in which the processor 110 automatically performs an additional task are as follows.

As an example, in the case where the degree of dispersion of the first group is less than a first threshold dispersion degree, the processor 110 may perform a first task of stopping analysis of a pathological slide image. In addition, the processor 110 may start a task for a subsequent action (e.g., cleaning or redyeing of a slide, or the like).

As another example, in the case where the degree of dispersion of the first group is greater than or equal to the first threshold dispersion degree and less than a second threshold dispersion degree, the processor 110 may perform a second task of analyzing the rest excluding a noise patch from the pathological slide image. In addition, the processor 110 may output a result of analysis. Here, the processor 110 may exclude the whole noise patch from an analysis target or may exclude only an artifact area from the analysis target by segmenting the artifact area included within the noise patch.

As another example, in the case where the degree of dispersion of the first group is greater than or equal to the second threshold dispersion degree, the processor 110 may perform a third task of analyzing the whole pathological slide image. In addition, the processor 110 may output the result of the analysis.

Here, the first threshold dispersion degree and the second threshold dispersion degree may be determined in advance, or may be set or adjusted by the user 30.

As another example, the processor 110 may provide data for the user 30 to determine a subsequent task by outputting together the result of analysis performed after excluding the noise patch from the pathological slide image (i.e., the result of the second task) and the result of analysis performed without excluding the noise patch (i.e., the result of the third task).

Subsequently, examples in which the processor 110 outputs information regarding an additional task are as follows. As the processor 110 outputs the information regarding the additional task, the user 30 may determine and perform the subsequent task.

As an example, in the case where the degree of dispersion of the first group is less than the first threshold dispersion degree, the processor 110 may notify that the stop of analysis of the pathological slide image and a subsequent action (e.g., cleaning or re-staining of a slide, or the like) are needed.

As another example, in the case where the degree of dispersion of the first group is greater than or equal to the first threshold dispersion degree, the processor 110 may output together the result of analysis performed after excluding the noise patch from the pathological slide image and the result of analysis performed without excluding the noise patch. In addition, in the case where a pathological result difference, which may affect decision-making, is present between the two results, the processor 110 may additionally guide the user into the same (e.g., output a warning or the like).

Meanwhile, although not illustrated in FIGS. 11A to 13B, the processor 110 may determine a type of an additional task according to a location of the first group in the sum of the first group and the second group.

In the case wherein a location of the noise patch in the pathology slide image is located within an area of interest (e.g., a cancer tissue, a cancer stromal tissue, or the like), accuracy of analysis of the pathological slide image may be lowered. Accordingly, as the processor 110 performs different subsequent procedures according to the location of the noise patch within the pathological slide image, the accuracy of analysis of the pathological slide image may increase.

The processor 110 determines the type of the additional task on the basis of the location of the first group (i.e., whether or not the noise patch is located within a tissue of interest). For example, the processor 110 may calculate a ratio of the number of noise patches located within the area of interest to the total number of noise patches, and determine the calculated ratio as the location of the first group. In addition, the processor 110 automatically performs the additional task or outputs information regarding the additional task.

First, examples in which the processor 110 automatically performs the additional task are as follows.

As an example, in the case where the calculated ratio exceeds a first threshold ratio, the processor 110 may perform a first task of stopping analysis of the pathological slide image. In addition, the processor 110 may start a task for a subsequent action (e.g., cleaning or re-staining of a slide or the like).

As another example, in the case where the calculated ratio is less than or equal to the first threshold ratio and exceeds a second threshold ratio, the processor 110 may perform a second task of analyzing the rest excluding the noise patch from the pathological slide image. In addition, the processor 110 may output the result of analysis. Here, the processor 110 may exclude the whole noise patch from an analysis target or may exclude only an artifact area from the analysis target by segmenting the artifact area included within the noise patch.

As another example, in the case where the calculated ratio is less than or equal to the second threshold ratio, the processor 110 may perform a third task of analyzing the whole pathological slide image. In addition, the processor 110 may output the result of analysis.

Here, the first threshold ratio and the second threshold ratio may be determined in advance, or may be set or adjusted by the user 30.

As another example, the processor 110 may provide data for the user 30 to determine a subsequent task by outputting together the result of analysis performed after excluding the noise patch from the pathological slide image (i.e., the result of the second task) and the result of analysis performed without excluding the noise patch (i.e., the result of the third task).

Subsequently, examples in which the processor 110 outputs the information regarding the additional task are as follows. As the processor 110 outputs information regarding an additional task, the user 30 may determine and perform a subsequent task.

As an example, in the case where the calculated ratio exceeds the first threshold ratio, the processor 110 may notify that the stop of analysis of the pathological slide image and a subsequent action (e.g., cleaning or re-staining of a slide or the like) are needed.

As another example, in the case where the calculated ratio is less than or equal to the first threshold ratio, the processor 110 may output together the result of analysis performed after excluding the noise patch from the pathological slide image and the result of analysis performed without excluding the noise patch. In addition, in the case where a pathological result difference, which may affect decision-making, is present between the two results, the processor 110 may additionally guide the user into the same (e.g., output a warning or the like).

FIG. 14 is a flowchart illustrating another example in which a processor performs an additional task, according to an embodiment.

In operation 1510, the processor 110 determines a type of an additional task according to a relationship between whether or not at least one patch having at least one artifact is removed and quality of analysis of a pathological slide image.

For example, the processor 110 may predict quality of analysis performed after excluding a noise patch from the pathological slide image and quality of analysis in the case where the whole pathological slide image including the noise patch is analyzed. Here, the quality of analysis may be determined on the basis of whether or not the result of analysis of an image affects a determination of treatment response. In addition, the processor 110 may determine the type of the additional task according to the result of the prediction.

In operation 1520, the processor 110 automatically performs the additional task or outputs information regarding the additional task.

For example, the additional task may include at least one of a first task of stopping analysis of the pathological slide image, a second task of analyzing the rest excluding at least one noise patch from the pathological slide image, and a third task of analyzing the whole pathological slide image. Examples of the first task to the third task are as described above with reference to operation 1020.

In the case where the processor 110 outputs the information regarding the additional task, the user 30 may determine whether or not to perform the additional task after identifying information provided by the processor 110.

Although not illustrated in FIGS. 10 and 14, after performing operation 1020 or operation 1520, in the case where the distribution of noise patches within the pathological slide image is determined to be statistically significant, the processor 110 may additionally provide the user 30 with information related to the distribution of the noise patches.

For example, information indicating that noise patches are intensively distributed on the left or right along the x-axis, information indicating that noise patches are intensively distributed at the top or bottom along the y-axis, information indicating that noise patches are intensively distributed on a particular quadrant, or the like may be provided to the user.

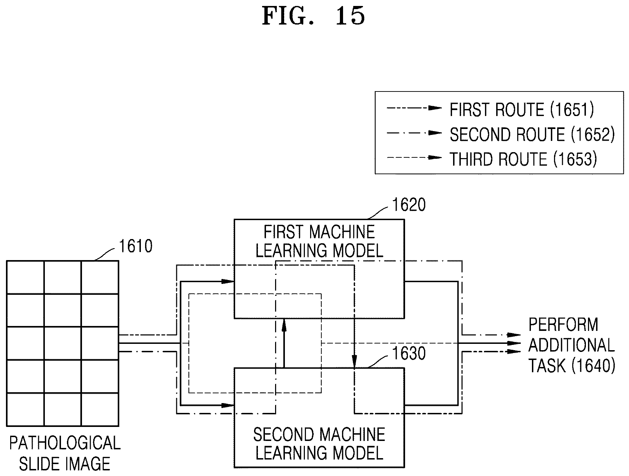

Meanwhile, the method described above with reference to FIGS. 1 to 14 may be performed by at least one machine learning model. Hereinafter, an example in which a method of analyzing a pathological slide image is performed by at least one machine learning model is described with reference to FIG. 15.

FIG. 15 is a view illustrating an example in which a method of analyzing a pathological slide image is performed by at least one machine learning model, according to an embodiment.

Referring to FIG. 15, at least one process of analyzing a pathological slide image 1610 may be performed by a first machine learning model 1620 and/or a second machine learning model 1630. For example, the first machine learning model 1620 may identify or divide a noise patch from the pathological slide image 1610. In addition, the second machine learning model 1630 may analyze the pathological slide image 1610 to output an inference result (e.g., a location, type, and class of a tissue, cell, protein and/or genome, an expression level of a biomarker, survival data of a patient, reactivity to a drug, or the like).

For convenience of description, the first machine learning model 1620 and the second machine learning model 1630 are separately illustrated in FIG. 15, but are not limited thereto. In other words, the first machine learning model 1620 and the second machine learning model 1630 may be implemented as a single machine learning model.

The method described above with reference to FIGS. 1 to 14 is described as including a first route 1651 in which the second machine learning model 1630 operates after the operation of the first machine learning model 1620 and thus an additional task is performed 1640. In particular, as described above, after the noise patch is identified by the first machine learning model 1620, all patches or some patches excluding the noise patch from among all patches are analyzed by the second machine learning model 1630 on the basis of the result of the identification and thus a medical inference result is output. However, the method described above with reference to FIGS. 1 to 14 is not limited to the first route 1651.

For example, the method described above with reference to FIGS. 1 to 14 may include a second route 1652 in which the first machine learning model 1620 operates after the operation of the second machine learning model 1630 and thus the additional task is performed 1640. In particular, after the pathological slide image 1610 is analyzed by the second machine learning model 1630, at least one noise patch from among all patches may be identified by the first machine learning model 1620 by referring to the result of analysis.

Alternatively, the method described above with reference to FIGS. 1 to 14 may include a third route 1653 in which the first machine learning model 1620 and the second machine learning model 1630 operate in parallel and thus the additional task is performed 1640. In particular, the first machine learning model 1620 and the second machine learning model 1630 may interact and operate simultaneously to output the results of analysis of at least some patches of the pathological slide image 1610.

Here, a machine learning model refers to a statistical learning algorithm implemented on the basis of a structure of a biological neural network in machine learning technology and cognitive science, or a structure that executes the algorithm.

For example, the machine learning model may refer to a model having a problem-solving capability as nodes that are artificial neurons forming a network with a combination of synapses as in a biological neural network are trained to reduce an error between a correct output corresponding to a particular input and an inferred output by repeatedly adjusting weights of the synapses. For example, the machine learning model may include any probability model, a neural network model, or the like used in an artificial intelligence learning method such as machine learning or deep learning.

For example, the machine learning model may be implemented as a multilayer perceptron (MLP) including multilayer nodes and connections between the multilayer nodes. The machine learning model according to the present embodiment may be implemented by using one of various artificial neural network model structures including MLPs. For example, the machine learning model may include an input layer that receives an input signal or data from the outside, an output layer that outputs an output signal or data corresponding to the input data, and at least one hidden layer that is located between the input layer and the output layer, receives a signal from the input layer, extracts features, and delivers the features to the output layer. The output layer receives a signal or data from the hidden layer and outputs the same to the outside.

Therefore, the machine learning model may be trained to receive one or more pathological slide images and extract information regarding one or more subjects (e.g., a cell, a tissue, a structure, and the like) included in the pathological slide images.

According to the above description, an additional analysis procedure may be differently performed by determining whether or not each of patches constituting a pathological slide image is a noise patch and considering information regarding a relationship between the noise patch and another patch rather than excluding the noise patch from an analysis target. In addition, as the identified noise patch is output, the noise patch may be added/changed/excluded through a determination by the user 30.

In addition, according to the above description, an area in which a subject is expressed may be identified, rather than a background area within the pathological slide image, and analysis may be performed within the identified area. In addition, the pathological slide image is analyzed by certain area, and thus, medical prediction may be performed by using even an image including an artifact.

Meanwhile, the above-described method may be written as a program that may be executed on a computer, and may be implemented in a general-purpose digital computer that operates the program by using a computer-readable recording medium. In addition, a structure of data used in the above-described method may be recorded in a computer-readable recording medium via various types of means. The computer-readable recording medium includes a storage medium, such as a magnetic storage medium (e.g., ROM, RAM, a USB, a floppy disk, a hard disk, or the like) and an optically readable medium (e.g., CD-ROM, DVD, or the like).

Those skilled in the art related to the present embodiment may understand that the present embodiment may be implemented in a modified form within the scope that does not depart from the essential characteristics of the above description. Therefore, the disclosed methods should be considered in an illustrative rather than a restrictive sense, and the scope of the present disclosure should be defined by claims rather than the foregoing description, and should be construed to include all differences within the scope equivalent thereto.

## Claims

1. A computing device comprising:
at least one memory; and
at least one processor, wherein the processor is configured to divide a pathological slide image into a plurality of patches, determine whether or not at least one artifact is present in at least one of the plurality of patches, and perform an additional task on the basis of a result of the determination.

2. The computing device of claim 1, wherein the processor is further configured to determine whether or not the at least one artifact is present in at least one patch needing to be analyzed, from among the plurality of patches, and the at least one patch needing to be analyzed comprises at least one of a patch comprising a subject, a patch comprising an area of interest, and a patch not comprising only a background area.

3. The computing device of claim 1, wherein the processor is further configured to output information regarding the plurality of patches reflecting the result of the determination, and update the result of the determination on the basis of a user input according to identification of the output information.

4. The computing device of claim 3, wherein the output of the information is performed according to at least one of a first type distinguishing the plurality of patches according to whether or not the at least one artifact is present and a second type distinguishing the plurality of patches into a patch needing to be analyzed, a patch that does not need to be analyzed, or a patch comprising the at least one artifact.

5. The computing device of claim 1, wherein the processor is further configured to determine a type of the additional task according to a relationship between a first group of patches in which the at least one artifact is present and a second group of patches in which the at least one artifact is not present.

6. The computing device of claim 5, wherein the processor is further configured to automatically perform a determined additional task or output information regarding a determined additional task.

7. The computing device of claim 5, wherein the relationship comprises at least one of a ratio occupied by the first group in a sum of the first group and the second group, a distribution of the first group in the sum of the first group and the second group, and a location of the first group in the sum of the first group and the second group.

8. The computing device of claim 1, wherein the processor is further configured to compare an analysis result obtained in a case where the whole pathological slide image is analyzed with an analysis result obtained in a case where analysis is performed after removing, from the pathological slide image, at least one patch in which the at least one artifact is present, and determine the type of the additional task on the basis of a result of the comparison.

9. The computing device of claim 1, wherein the additional task comprises at least one of a first task of stopping analysis of the pathological slide image, a second task of analyzing the rest excluding at least one patch comprising the at least one artifact from the pathological slide image, and a third task of analyzing the whole pathological slide image.

10. A method of analyzing a pathological slide image, the method comprising:
dividing a pathological slide image into a plurality of patches;
determining whether or not at least one artifact is present in at least one of the plurality of patches; and
performing an additional task on the basis of a result of the determination.

11. The method of claim 10, wherein the determining comprises determining whether or not the at least one artifact is present in at least one patch needing to be analyzed, from among the plurality of patches, and the at least one patch needing to be analyzed comprises at least one of a patch comprising a subject, a patch comprising an area of interest, and a patch not comprising only a background area.

12. The method of claim 10, wherein the performing comprises:
outputting information regarding the plurality of patches reflecting a result of the determination; and
updating the result of the determination on the basis of a user input according to identification of the output information.

13. The method of claim 12, wherein the outputting is performed according to at least one of a first type distinguishing the plurality of patches according to whether or not the at least one artifact is present and a second type distinguishing the plurality of patches into a patch needing to be analyzed, a patch that does not need to be analyzed, or a patch comprising the at least one artifact.

14. The method of claim 10, wherein the performing comprises determining a type of the additional task according to a relationship between a first group of patches in which the at least one artifact is present and a second group of patches in which the at least one artifact is not present.

15. The method of claim 14, further comprising automatically performing a determined additional task or outputting information regarding a determined additional task.

16. The method of claim 14, wherein the relationship comprises at least one of a ratio occupied by the first group in a sum of the first group and the second group, a distribution of the first group in the sum of the first group and the second group, and a location of the first group in the sum of the first group and the second group.

17. The method of claim 10, wherein the performing comprises:
comparing an analysis result obtained in a case where the whole pathological slide image is analyzed with an analysis result obtained in a case where analysis is performed after removing, from the pathological slide image, at least one patch in which the at least one artifact is present; and determining the type of the additional task on the basis of a result of the comparison.

18. The method of claim 10, wherein the additional task comprises at least one of a first task of stopping analysis of the pathological slide image, a second task of analyzing the rest excluding at least one patch comprising the at least one artifact from the pathological slide image, and a third task of analyzing the whole pathological slide image.

19. A computer-readable recording medium having recorded thereon a program for causing a computer to execute the method of claim 10.
